# EUROPÄISCHE PATENTSCHRIFT

(11) **EP 3 017 052 B1**
(45) Veröffentlichungstag und Bekanntmachung des Hinweises auf die Patenterteilung: **23.08.2017**
(21) Anmeldenummer: 15733641.3
(22) Anmeldetag: 10.06.2015
(51) Int. Cl.: C12P 5/02, C12P 7/10, C12P 7/54

(54) **VERFAHREN ZUR INITIALISIERUNG DES FERMENTATIONSPROZESSES IN BIOGASANLAGEN**
METHOD FOR INITIALIZING THE FERMENTATION PROCESS IN BIOGAS PLANTS
PROCÉDÉ D'INITIALISATION DU PROCESSUS DE FERMENTATION DANS DES INSTALLATIONS DE PRODUCTION DE BIOGAZ

(30) Priorität: 12.06.2014 DE 102014108233
(43) Veröffentlichungstag der Anmeldung: 11.05.2016
(73) Patentinhaber: Rabe, Petra, 15713 Königs Wusterhausen (DE)
(72) Erfinder: RABE, Petra, 15713 Königs Wusterhausen (DE)
(74) Vertreter: Gulde & Partner
(86) Internationale Anmeldenummer: PCT/EP2015/062941
(87) Internationale Veröffentlichungsnummer: WO 2015/189272

(56) Entgegenhaltungen:
- WO-A1-2013/185777
- Anonymous: "Verfahren, die Avantec-Hydrolyse", Avantec Biogas DE , 30. Juli 2013 (2013-07-30), XP002751254, Gefunden im Internet: URL:http://web.archive.org/web/20130730111 710/http://avantec-biogas.de/verfahren.htm l [gefunden am 2015-11-23] in der Anmeldung erwähnt
- SUÁREZ QUIÑONES, T. ET AL.: "Hydrolytic Enzymes Enhancing Anaerobic Digestion", Biogas Production: Pretreatment Methods in Anaerobic Digestion, Mai 2012 (2012-05), Seiten 157-198, XP055108763, DOI: 10.1002/9781118404089.ch6 Gefunden im Internet: URL:http://onlinelibrary.wiley.com/store/1 0.1002/9781118404089.ch6/asset/ch6.pdf?v=1 &t=hsxdx2lg&s=593ec6ba5b9713dcd93309073f4e 00cf09013335
- Anonymous: "Bionova-Biogas - Technologie", Bionova Biogas , 11. August 2015 (2015-08-11), XP002751255, Gefunden im Internet: URL:http://bionova-biogas.de/technologie.h tml [gefunden am 2015-09-15]

## Beschreibung

Die Erfindung betrifft ein Verfahren zur Initialisierung des Fermentationsprozesses in Biogasanlagen umfassend Vorfermenter und Methanfermenter unter Verwendung biologisch schwer abbaubarer organischer Stoffe, wobei diese in einem semi-aeroben Vorfermentationsschritt so aufgeschlossen werden, dass sie für die Erzeugung von Biogas in anaeroben Fermentern verfügbar werden. Unter schwer abbaubaren organischen Rohstoffen werden Reststoffe aus der Landwirtschaft, der Landschaftspflege und dem Landschaftsbau verstanden, die in der Regel stabile Faserstrukturen aufweisen, wie Gras, Heu, Stroh (einschließlich Mais- und Reisstroh), Schilf, Dung usw. Die Faserfraktion der Rohstoffe enthält größtenteils Cellulose, jedoch auch Anteile an Hemicellulosen und Lignin.

Durch einen mehrstufigen Initialisierungsprozess wird durch eine spezielle Prozessführung effektiv Biogas erzeugt, wobei in einem Vorfermenter eine komplexe Biozönose aufgebaut wird, die in der Lage ist, die stabilen, Faserstrukturen bildenden organischen Verbindungen aufzulösen und in für die Biogasbildung geeignete organische Säuren und niedermolekulare Verbindungen umzuwandeln.

### Stand der Technik

Biogas ist ein Gasgemisch, das beim Abbau organischer Stoffe unter Luftausschluss (anaerob) entsteht und sich im Wesentlichen aus Methan und Kohlendioxid zusammensetzt. Das enthaltene Methan ist brennbar und wird als Energieträger zur Erzeugung von Wärme und Elektroenergie genutzt.

Die methanbildenden Mikroorganismen leben streng anaerob und gehören zu den Archaebacter. Unterschiedliche Spezies verstoffwechseln entweder Essigsäure zu Methan und Kohlendioxid oder Kohlendioxid und Wasserstoff zu Methan.

Um aus den natürlich vorhandenen organischen Materialien, Fetten, Kohlenhydraten und Proteinen, die Ausgangsstoffe für die Methanbakterien zu erhalten, sind mehrere vorgelagerte Abbauschritte durch andere Mikroorganismen erforderlich. Zunächst werden langkettige, in Wasser unlösliche organische Moleküle durch enzymatischen Aufschluss in wasserlösliche Bausteine aufgespalten (Hydrolyse). Dazu sind Exo-Enzym bildende Spezies in der Lage. Säurebildende Bakterien verstoffwechseln diese wasserlöslichen organischen Verbindungen zu organischen Säuren (Acidogenese). Dabei wird zum Teil Essigsäure gebildet, die den Methanbakterien zur Verfügung steht. Andere Fettsäuren und Milchsäure werden teilweise durch acetogene Bakterien in Essigsäure umgewandelt (Acetogenese) oder zu Wasserstoff und Kohlendioxid weiter abgebaut.

In traditionellen Biogasanlagen finden alle Abbauschritte bis zur Methanbildung in einem Fermenter statt, der unter Luftabschluss arbeitet. Um Prozessstörungen durch die unterschiedlichen Regenerationszeiten und Prozessoptima der verschiedenen Bakterienkulturen zu vermeiden, werden die Fermenter mit geringen organischen Belastungen betrieben.

Bei der Verwendung von leicht abbaubaren Einsatzstoffen wie zum Beispiel Fetten oder Lebensmittelabfällen werden die beiden ersten Prozessstufen Hydrolyse und Acidogenese häufig in einem getrennten Fermenter durchgeführt, der üblicherweiseinsgesamt als Hydrolyse bezeichnet wird. Damit wird vermieden, dass die bei niedrigen pH-Werten mit kurzen Regenerationszeiten arbeitenden Bakterien dieser Stufen zu einer Übersäuerung und einem Säurestau im Fermenter führen, der die Methanbildung hemmen würde.

DE 296 05 625 U1 beschreibt eine Anlage zur Vergärung von organischen Abfallstoffen in der dem Fermenter eine semi-aerobe Hydrolysestufe vorgeschaltet ist. Diese wird in Abhängigkeit vom Gärsäurespektrum kontinuierlich belüftet und bei Temperaturen im Bereich von 20 bis 40 °C betrieben. Zum Animpfen der Prozessbiologie in Hydrolyse und Methanisierung wird der flüssige Teil des vorher in Absetzbehältern separierten Gärrestes genutzt. Dieses Verfahren eignet sich jedoch nicht für Kohlenwasserstoff-haltige, schwer abbaubare Stoffe. Hier ist zum Aufschluss der langkettigen Moleküle ein hoher Sauerstoffeintrag erforderlich, der in der Folge gleichzeitig zum aeroben Abbau der gebildeten Zucker und Stärken führt, wodurch das zur Biogasbildung nutzbare Material verbraucht wird.

In letzter Zeit rückt die Nutzung faserhaltiger Reststoffe aus der Landwirtschaft, der Landschaftspflege und dem Landschaftsbau, die für die menschliche und Tierernährung nicht nutzbar sind, zunehmend in den Fokus der Biogasgewinnung. Eine Schwierigkeit bei deren Vergärung besteht darin, dass die für die Biogasgewinnung nutzbaren organischen Stoffe in anaerob biologisch kaum abbaubaren Gerüststrukturen aus Lignocellulosen, Cellulosen, Kieselsäuren oder Keratinen eingebettet sind.

Zur Lösung dieses Problems wird in DE 10 2007 012 104 A1 ein Verfahren zum Hybridaufschluss von lignocellulosehaltiger Biomasse vorgeschlagen, bei dem die Faserstrukturen zunächst durch Extrusion thermo-mechanisch aufgeschlossen werden und in einem zweiten Schritt die Lignocellulose-Komplexe thermisch durch Mikrowellen und gegebenenfalls unter Zusatz von Enzymen aufgebrochen werden. Dieses Verfahren ist mit einem hohen apparativen und energetischen Aufwand verbunden und benötigt den Zusatz von Enzymen um zusätzlich zu den in den Zellen enthaltenen leichter abbaubaren organischen Stoffen auch die Cellulosen und Hemicellulosen für die Methanbildung nutzen zu können.

DE 10 2008 007 423 A1 stellt ein Verfahren zur Erzeugung von Biogas in zumindest zwei Schritten vor, bei dem dem Fermenter eine anaerobe Hydrolysestufe in Form eines Pfropfenstromfermenters vorgeschaltet ist. Die Besonderheit des Verfahrens besteht darin, dass der Hydrolysestufe ein Anteil aus Gärmaterial der Methanisierungsstufe zugesetzt wird und damit der Anteil von Methan im Hydrolysegas auf 10 bis 30 % erhöht wird. Dieses Gas wird thermisch zum Aufheizen des Fermenters genutzt, so dass der gesamte energetische Wirkungsgrad der Umsetzung der Organik in Methan erhöht wird.

DE 10 2008 015 609 A1 beschreibt eine Biogasanlage und ein Verfahren zur Erzeugung von Biogas in einem mehrstufigen Prozess, welche zumindest zwei Hydrolysen und einen Fermenter zur Methanbildung umfassen. Die beiden dem Fermenter vorgeschalteten Hydrolysen werden wechselweise im Batch-Verfahren betrieben, das heißt, es wird jeweils eine vollständig entleerte Hydrolyse mit den für die Biogasproduktion genutzten Rohstoffen beschickt und mit aus dem Gärrest des Fermenters abgepresster Flüssigkeit soweit angemischt, dass die Masse rührfähig ist. Dieser Ansatz wird auf eine Temperatur zwischen 30 und 60 °C aufgeheizt und zwei bis vier Tage unter Rühren hydrolysiert und versäuert. Nach Abschluss dieser Zeit, wird das so erzeugte Hydrolysat für die Beschickung des Fermenters zur Verfügung gestellt. Der inzwischen vollständig entleerte zweite Hydrolysebehälter wird dann in derselben Weise wie vorher der erste beschickt und hydrolysiert.

Die in den beiden vorgehend beschriebenen Verfahren genutzten Hydrolysen weisen den Nachteil auf, dass sie anaerob betrieben werden. Es gibt jedoch nur eine geringe Zahl anaerober enzymbildender Bakterien, die Cellulasen produzieren können und das in ausreichender Menge und Qualität, so dass bei diesen Hydrolysen, die strukturbildenden Lignocellulosen im besten Fall in geringem Maße für die Biogasbildung genutzt werden können.

In der Zeitschrift Forum der Wissenschaft, Jahrgang 10, Nr. 19, 2006, S. 63-68 ist ein zweistufiges fest-flüssig Biogasverfahren mit offener Hydrolyse - ein neues technologisches Konzept für die Biogasgewinnung aus nachwachsenden Rohstoffen und bioverfügbaren Abfällen beschrieben. Hier werden die faserreichen Einsatzstoffe mit dem vorher belüfteten flüssigen Anteil des Gärrestes nach Abtrennen der Feststoffe angemischt und in einem offenen Behälter hydrolysiert. Im Behälter wird ein sogenanntes Bioleaching durchgeführt bei dem die festen Stoffe von der Flüssigkeit gegebenenfalls unter Zusatz von Enzymen durchspült werden. Die während der Hydrolyse aus den festen Rohstoffen ausgelösten bioverfügbaren organischen Substanzen werden mit der Flüssigkeit in einen Hydrolysatbehälter gefördert. Dort stehen sie zur Methanisierung in einem Hochleistungsfermenter mit immobilisierten Methanbakterien zur Verfügung. Eine gezielte Verfahrensführung hinsichtlich der Sauerstoffversorgung der Hydrolyse ist mit diesem Verfahren nicht zu erreichen.

Das in DE 10 2010 010 091 A1 beschriebene Bioliquid-Verfahren umfasst insgesamt zehn Prozessstufen. Hier werden die faserhaltigen Einsatzstoffe zunächst gewaschen und von Störstoffen befreit. Anschließend werden sie durch einen Extruder zerkleinert wobei auch Flüssigkeit abgeschieden wird. Die erhaltene Fasermischung durch Zufuhr von Warmluft einem aeroben Rotteprozess unterzogen. Gleichzeitig wird sie mittels der in einem Flüssigkeitstank gesammelten Flüssigkeit aus dem Extruder und nachgelagerten Separationsstufen, die bei Bedarf mit Enzymen und Harnstoff angereichert wird, durchrieselt. Dabei werden die gelösten organischen Stoffe aus der Rotte gewonnen und zusammen mit einem Teil des bereits gerotteten Materials einer Versäuerung (hier Hydrolyse genannt) zugeführt. Nach der Säurebildung werden die verbliebenen Fasern erneut durch eine Schneckenpresse abgetrennt. Die feuchten Feststoffe werden gepresst und getrocknet und können als Heizmittel Verwendung finden. Die abgetrennte mit organischen Säuren angereicherte Flüssigkeit wird in einem Methanfermenter zu Biogas vergoren. Dieses Verfahren ist technisch und energetisch mit erheblichem Aufwand verbunden. Aufgrund der in der Rotte herrschenden Milieubedingungen können Enzym bildende Bakterien nicht in ausreichendem Maße aktiv werden, so dass vorgesehen ist, der Rotte zusätzlich Enzyme zuzuführen.

WO 2013/185777 A1 offenbart ein Verfahren zur Initialisierung des Fermentationsprozesses in Biogasanlagen, die Vorfermenter und Methanfermenter aufweisen, unter Verwendung organischer Rohstoffe. Auch SUÁREZ QUIÑONES, T. ET AL., offenbaren in "Hydrolytic Enzymes Enhancing Anaerobic Digestion", Biogas Production: Pretreatment Methods in Anaerobic Digestion, doi:10.1002/9781118404089.ch6, (201205), Seiten 157 - 198, ein Verfahren zur Herstellung von Biogas unter Verwendung biologisch schwer abbaubarer faseriger organischer Reststoffe aus der Landwirtschaft, die stabile Faserstrukturen aufweisen, Seit 2005 wurde ein semi-aerobes Hydrolyseverfahren mehrfach veröffentlicht, das die vorgenannten Nachteile nicht aufweist, z. B. Tagung Biogaz Methanisation, Journees rencontres «Recherche et Industrie», INSA de Lyon, 08.02.2012, P. Rabe, C. Terrasse, Research Status and Practice of Semi-Aerobic Hydrolysis as a Pre-Digestive StepofOrganic Substrates in Biogas Plants, www.bionova.de/index.php/hydrolyse/verfahren, www.avantec-biogas.de/verfahren.html. Bei diesem zweistufigen Biogasverfahren ist dem Fermenter eine intervallweise belüftete Hydrolysestufe vorgeschaltet, die sowohl im Batch mit täglich einer Beschickung als auch kontinuierlich mit täglich mehrfacher Beschickung im Temperaturbereich zwischen 25 und 42 °C bei Verweilzeiten zwischen 0,5 und zwei Tagen betrieben wird. Durch Wahl der Belüftungsintensität, der Belüftungsintervalle und der Temperatur im vorgeschalteten Hydrolysefermenter gelingt es, dort eine Biozönose langfristig zu etablieren, durch die ein stabiler dreistufiger biologischer Prozess etabliert wird. In dessen erstem aeroben Schritt spalten natürliche im Rohstoff bereits vorhandene Enzym bildende Mikroorganismen die im Fasermaterial enthalten Lignocellulose-Strukturen auf und wandeln Cellulosen und HemiCellulosen in Zucker bzw. Mehrfachzucker um. Im anschließenden unbelüfteten anaeroben Intervall werden diese durch Bakterien, z.B. Hefen oder Milchsäurebakterien, die ebenfalls auf natürlichem Weg in die Hydrolyse gelangen, in Ethanol, Essigsäure und Milchsäure umgewandelt. Auf untergeordneten Nebenwegen werden in dieser Phase durch anaerobe Säurebildner in geringem Maße auch weitere Gärsäuren gebildet. In der anschließenden aeroben Phase wird der gebildete Alkohol durch aerobe Acetobacter zu Essigsäure verstoffwechselt. Die Hydrolyseabluft enthält als Hauptbestandteile neben dem aus der Zuluft stammenden Stickstoff, den nicht verbrauchten Sauerstoff und Kohlendioxid. Methan, Schwefelwasserstoff und Wasserstoff treten nur im ppm-Bereich der Konzentration auf. Eine Abluftreinigung über ein Biofilter ist empfehlenswert, da die Abluft geruchsbelastet ist. Dieses Verfahren zeichnet sich durch eine hohe Effizienz hinsichtlich der Erhöhung der erreichbaren Biogasausbeuten sowie eines relativ geringen apparativen und energetischen Aufwandes aus. Das Verfahren wurde seit 2004 bereits in mehr als dreißig Biogasanlagen sowohl in Deutschland als auch in Italien, Frankreich, Tschechien, Lettland und Weißrussland erfolgreich zur Vergärung von faserreichen Grassilagen, Maissilagen und Mist aus der Tierhaltung eingesetzt. Ein Nachteil des Verfahrens besteht jedoch darin, dass bei der Vergärung vorwiegend trockener, wenig eigene Bakterienkulturen enthaltender faserhaltiger organischer Stoffe, wie z. B. Landschaftspflegematerial von Wiesen, Uferrändern, von Treibgut (vorwiegend Schilf), von Stroh, Maisstroh und Reisstroh, eine für die semi-aerobe günstige Biozönose aus dem Material heraus nicht mit genügender Sicherheit gelingt.

Der Erfindung lag deshalb die Aufgabe zugrunde, faserreiche Roh- und Reststoffe, welche in großem Maße zur Verfügung stehen, so aufzubereiten, dass sie kostengünstig und effektiv zur Fermentation und Herstellung von Biogas geeignet sind.

Mit Hilfe des vorliegenden Verfahrens wird diese Aufgabe gelöst. Im Sinne der Erfindung werden unter biologisch schwer abbaubaren faserigen organischen Rohstoffen Reststoffe aus der Landwirtschaft, die stabile Faserstrukturen aufweisen, verstanden. Überraschenderweise hat sich gezeigt, dass es möglich ist, eine stabile Biozönose in einer semi-aeroben Vorfermentation zu etablieren, die in der Lage ist sowohl die notwendigen aeroben Verfahrensstufen der Enzymbildung und Essigsäurebildung als auch die anaerobe Ethanolbildung als Voraussetzung der Essigsäurebildung unter den wechselnden Bedingungen im Vorfermenter (so genannte Hydrolyse) zu etablieren. Erfindungsgemäß wird ein Verfahren zur Initialisierung des Fermentationsprozesses für Biogasanlagen bereitgestellt, die Vorfermenter und Methanfermenter aufweisen. Das erfindungsgemäße Aufschlussverfahren ermöglicht eine effiziente Weiterverarbeitung der Hydrolysate zu den jeweiligen Produkten, wobei die hydrolysierte Biomasse zur Erzeugung von Biogas bei beträchtlich gesteigertem Biogasertrag eingesetzt werden kann. Durch das Verfahren wird eine weitgehend strukturelle Desintegration schwer abbaubarer, lignifizierter Biomassen ermöglicht.

Erfindungsgemäß wird in einem ersten Verfahrensschritt im Vorfermenter die Biomasse, der zu vergärende faserreiche organische Rohstoff, in einer Flüssigkeit eingeweicht, so dass er im Vorfermenter dickflüssig, aber rührfähig ist. Der Trockensubstanzgehalt beträgt maximal 20 %, so dass ein rührfähiger Brei entsteht. Sehr langfaserige Materialien, wie Gras oder Maisstroh, werden mit einer Flüssigkeit so vermischt, dass sie mit einem Trockensubstanzgehalt von 9 bis 11 % im Vorfermenter vorliegen.

Unter sehr langfaserigen Materialien werden z. B. Rohstoffe aus der Landwirtschaft, der Landschaftspflege und dem Landschaftsbau verstanden, die noch nicht angegoren sind, wie Gras, Heu oder Stroh, z. B. Weizen-, Mais- oder Reisstroh oder auch Schilf. Werden im erfindungsgemäßen Verfahren angerottete Materialien, deren Fasern dadurch bereits brüchig sind, als Rohstoffe verwendet, können diese bis auf 20 % Trockensubstanzgehalt in der Mischung angerührt werden.
Zum Einweichen/Anmischen kann Wasser oder eine andere vorhandene Flüssigkeit verwendet werden. Vorzugsweise werden zur Einstellung des Feststoffgehaltes neben Wasser Gülle und/oder Gärreste eingesetzt. In einer bevorzugten Ausführungsform werden Gülle oder Gärrest aus einer anderen Biogasanlage zum Anmischen verwendet, die aufgrund ihrer Konsistenz bereits eine breiige Struktur aufweisen und so in der Lage sind, eine Separation der Fasern von der Flüssigkeit zu behindern.

In einer weiteren Ausführungsform können zu Beginn des Prozesses weitere organische Materialien, vorzugsweise Cellulosederivate, z. B. Carboxyl-Methyl-Cellulosen, zum Andicken der Flüssigkeit, zugegeben werden. Carboxymethylcellulose ist als bewährtes Bindemittel bzw. Verdicker bekannt.

Nach dem Anmischen wird die Flüssigkeit im Vorfermenter unter Rühren auf eine Temperatur im Bereich zwischen 25 und 42 °C erwärmt. Die optimale Temperatur kann in Vorversuchen ermittelt werden. Diese festgelegte Temperatur wird über den gesamten Zeitraum der Verfahrensdurchführung beibehalten. Ist die für den verwendeten Rohstoff optimale Temperatur erreicht, wird der Mischung im Vorfermenter durch Einschalten einer Belüftung kontinuierlich Luft zugeführt. Die faserigen Rohstoffe, insbesondere trockene Materialien wie Heu oder Stroh, werden so angelöst.

Weiterhin wird die Mischung mit einer bekannten enzymbildenden Kultur aus Bakterien oder Pilzen beimpft. Diese Kulturen sind in der Lage Enzyme zu bilden, die für die Behandlung des Rohstoffes und zum enzymatischen Aufschluss benötigt werden. Es handelt sich um enzymproduzierende mikrobielle Rein- oder Mischkulturen, welche an die Zielsubstrate angepasst sind. Dem Fachmann sind diese Kulturen bekannt, im Wesentlichen handelt es sich bei den Enzymen um an sich bekannte Endo- und Exocellulasen sowie Glucosidasen.

Die Kultivierung unter kontinuierlicher Belüftung mit den enzymbildenden Mikroorganismen dauert etwa 1 bis 2 Tage. Vorzugsweise wird die Mischung bei konstanter Temperatur unter Rühren und Belüften 24 Stunden kultiviert. Der pH-Wert liegt im bevorzugten Bereich von 4 bis 8,2. In diesem Bereich liegt der pH-Wert während der gesamten Verfahrensdurchführung. Im Anschluss beginnt die erste Entnahme von hydrolysiertem Material aus dem Vorfermenter. Dieses kann bereits dem Methanfermenter zur Biogasbildung zugeführt werden. Voraussetzung ist, dass im Methanfermenter im Vorfeld bereits ein Methanbildungsprozess etabliert wurde. Dieses kann durch Befüllen mit Gülle, bevorzugt Rindergülle, oder Gärrest aus einer benachbarten Biogasanlage geschehen sein.
Die Entnahme aus dem Vorfermenter erfolgt in der durch die vorgesehene Technologie im Methanfermenter vorgegebenen Menge: entweder bei Batch-Betrieb die gesamte Tagesration oder bei kontinuierlichem Betrieb der vorgesehene Anteil an der Gesamt-Tagesration. Nach der Entnahme wird erneut frisches Material und Flüssigkeit im zu Beginn festgelegten Verhältnis in den Vorfermenter zugegeben. Weiterhin wird erneut eine Tagesration der enzymbildenden Kultur zugegeben und diese unter durchgehender Belüftung, Temperierung und Rühren kultiviert.

Dieses Vorgehen wird mehrfach, vorzugsweise 3- bis 5-mal, wiederholt, d. h. bevorzugt etwa 3 bis 5 Tage fortgeführt. Das hydrolisierte Material wird jeweils nach 1 bis 2 Tagen Kultivierung entnommen, dem Methanfermenter zugeführt und durch frisches Material ersetzt.
Nach einer Kultivierung von vorzugsweise ca. 5 bis 10 Tagen (in welchen immer wieder hydrolysiertes Material entnommen werden kann und frisches Material dem Vorfermenter zugeführt wird) wird im Anschluss an die Kultivierung in einem zweiten Verfahrensschritt die zugegebene Menge an enzymbildender Kultur im Vorfermenter auf 25 bis 50 % reduziert.
Gleichzeitig wird die Belüftung auf intervallweise umgestellt. Die Länge der belüfteten und unbelüfteten Intervalle wird so gewählt, wie sie im endgültigen technologischen Ablauf für den eingesetzten Rohstoff festgelegt wurde. Die Festlegung der Länge des Belüftungsintervalls erfolgt auf der Basis von Erfahrungen mit ähnlichen Substraten oder im Ergebnis von Vorversuchen. Im weiteren Verlauf des Verfahrens bleibt das Belüftungsintervall in gleicher Länge bestehen.
Gleichzeitig wird mit der Zugabe von geeigneten Produktionsorganismen in den Vorfermenter begonnen. In der Regel handelt es sich um Ethanol-bildende Kulturen. Das können z. B. Reinkulturen für die Ethanolproduktion sein oder auch heterofermentative Milchsäurebakterien oder Sauerteighefen. In dieser Betriebsweise wird der Vorfermenter weitere 3 bis 5 Tage betrieben. Im dritten Verfahrensschritt erfolgt keine Zugabe der enzymbildenden Kultur mehr und die Zugabe der Ethanol-bildenden Kultur wird auf 25 bis 50 % reduziert. Dieser Verfahrensschritt wird 3 bis 10 Tage durchgeführt. Wurde im zweiten Verfahrensschritt eine Reinkultur für die Ethanolbildung eingesetzt, wird mit Beginn des dritten Verfahrensschrittes eine Essigsäure produzierende Kultur implementiert.
Nach Beendigung des dritten Verfahrensschrittes kann die Implementierung der Essigsäure bildenden Kultur im vierten Verfahrensschritt mit auf 25 bis 50 % reduzierter Inkubationsmenge über drei bis sieben Tage fortgeführt werden.
Auf Grund der jeweiligen Restgehalte an C5-Zuckern in der Faserfraktion und C6-Zuckern im wässrigen Reaktionsüberstand ist es von großer Bedeutung, Mikroorganismen zu verwenden, die möglichst parallel beide Zuckerformen verwerten können.
Es wird eine vollständige stoffliche Nutzung mit hohen Produktausbeuten und -raten zur Herstellung von Milch- und Essigsäure angestrebt. Essigsäure produzierende Kulturen sind deshalb vorzugsweise Acetobacter. Dem Vorfermenter wird laufend hydrolysiertes Material entnommen und frisches Material zugeführt.
Nach Überführung in den Methanfermenter werden durch methanogene Archaeen aus Kohlenstoffdioxid, Wasserstoff und Essigsäure die Endprodukte Methan (CH₄) und Wasser gebildet.

### Das Verfahren hat die folgenden Vorteile:

Stabile Biogasproduktion und Erzielung hoher Konversionsraten von über 60 %. Vermeidung von Schaumbildung im Fermenter.

Einsatz von biologisch schwer abbaubaren organischen Stoffen wie Stroh, Dung und Schilf, Heu, Gras, Grassilagen.

### Beispiel:

Eine Biogasanlage besteht aus einer vorgeschalteten Hydrolyse mit 150 m³ Flüssigkeitsvolumen und 2.160 m³ Fermenterinhalt. In ihr sollen Rindergülle, Maissilage und Grassilage vergoren werden. Da nur wenig Gülle zur Verfügung steht, ist vorgesehen Gärrest aus dem Fermenter zum Anmischen der Substrate in der Hydrolyse einzusetzen.

Der Fermenter wird mit Gärrest aus einer naheliegenden Biogasanlage befüllt und auf Fermentationstemperatur aufgeheizt. Zu diesem Zeitpunkt wird die Hydrolyse mit 150 m³ der vorgesehenen Mischung aus Rindergülle, Maissilage, Grassilage und Gärrest befüllt. Die Mischung wird innerhalb eines Tages auf die vorgesehene Temperatur von 38 °C aufgeheizt. Anschließend wird am 1. Tag des Anfahrprozesses der Mischung 2 kg einer Trockenkultur von enzymbildenden Mikroorganismen zugegeben. Die Belüftung wird eingeschaltet und der Prozess in der Hydrolyse bei einer konstanten Temperatur von 38 °C unter ständigem Rühren durchgeführt.

Am 2. Tag werden 120 m³ Material aus der Hydrolyse in den Fermenter überführt und die notwendige Gärrestmenge für 120 m³ Substratmischung aus dem Fermenter entnommen. Die Hydrolyse wird mit 120 m³ frischer Substratmischung befüllt und es werden wiederum 2 kg Trockenkultur zugegeben. Unter ständigem Rühren und Belüften wird der Prozess weiter bei 38 °C fortgeführt.

Die tägliche Entnahmemenge aus dem Fermenter und Zugabe der Substratmischung in die Hydrolyse sowie die Temperatur in der Hydrolyse bleiben während des gesamten Inbetriebnahmezeitraumes konstant.

Am 3. und 4. Tag erfolgt die Belüftung jeweils intervallweise so, dass sie für 30 Minuten eingeschaltet und für 15 Minuten ausgeschaltet ist. Während der Belüftung wird der Rührer eingeschaltet. Es werden täglich 1 kg Enzymkulturen zugesetzt und zusätzlich 150 g einer heterofermentativen Milchsäure-Trockenkultur.

Vom 5. bis 9. Tag bleibt das Belüftungs- und Rührintervall wie am 3. und 4. Tag. Die Zugabe an Enzymkultur bleibt weiterhin bei 1 kg pro Tag. Die Zugabe von Milchsäurekultur wird auf 75 g pro Tag reduziert.

Vom 10. bis 14. Tag erfolgt keine Zugabe von Enzymkultur mehr. Die Belüftung und Rührung werden auf ein Intervall von 15 Minuten ein und 15 Minuten aus eingestellt. Die Zugabe von Milchsäurekultur bleibt weiterhin bei 75 g pro Tag.

Ab dem 15. Tag wird die Hydrolyse ohne Zusatz von Mikroorganismenkulturen im Belüftungs- und Rührintervall 15 Minuten ein und 15 Minuten aus bei 38 °C mit gleich bleibender Substratmischung und Zugabe in den Fermenter weiter betrieben.

## Patentansprüche

1. Verfahren zur Initialisierung des Fermentationsprozesses in Biogasanlagen, die Vorfermenter und Methanfermenter aufweisen, unter Verwendung biologisch schwer abbaubarer faseriger organischer Reststoffe aus der Landwirtschaft, die stabile Faserstrukturen aufweisen, umfassend die folgenden Schritte im Vorfermenter:
a) Einweichen der Rohstoffe in einer Flüssigkeit und Erwärmen auf eine Temperatur von 25 bis 42 °C, wobei der Trockensubstanzgehalt maximal 20 % beträgt, so dass ein rührfähiger Brei entsteht,
b) nach Erreichen der Reaktionstemperatur Zugabe von enzymbildenden Kulturen aus Bakterien oder Pilzen und 1 bis 2 Tage Kultivierung unter kontinuierlicher Belüftung,
c) erste Überführung von hydrolysiertem Material aus dem Vorfermenter in den Methanfermenter und erneute Zugabe von Rohmaterial und Flüssigkeit, wobei der Trockensubstanzgehalt bei maximal 20 % gehalten wird,
d) mehrfache Wiederholung von Schritt a) bis c),
e) anschließend Drosselung der Zugabe der enzymbildenden Kulturen auf 25 bis 50% sowie Zugabe von Produktionsorganismen für Ethanolbildung und Kultivierung für 3 bis 5 Tage unter intervallweiser Belüftung,
f) danach Stopp der Zugabe von enzymbildenden Kulturen und Drosselung der Zugabe der Ethanolbildungsorganismen auf 25 bis 50 % und Kultivierung für 3 bis 10 Tage ggf. unter Zugabe von Produktionsorganismen für die Essigsäurebildung,
wobei als enzymbildende Kulturen Mikroorganismen verwendet werden, die an sich bekannte Endo- und Exocellulasen sowie Glucosidasen bilden oder aufweisen.

2. Verfahren nach Anspruch 1, **gekennzeichnet durch** Drosselung der Zugabe der Essigsäurebildungsorganismen auf 25 bis 50 % und weitere Kultivierung für maximal 7 Tage.

3. Verfahren nach Anspruch 1 oder 2, **gekennzeichnet dadurch, dass** bei Verwendung langfaseriger Rohstoffe der Trockensubstanzgehalt auf 9-11% eingestellt wird.

4. Verfahren nach einem der Ansprüche 1 bis 3, **gekennzeichnet dadurch, dass** die biologisch schwer abbaubaren faserigen organischen Reststoffe aus der Landwirtschaft Gras, Grassilagen, Heu, Stroh, Schilf, Dung sind.

5. Verfahren nach einem der Ansprüche 1 bis 4, **gekennzeichnet dadurch, dass** die Flüssigkeiten zum Einweichen der Rohstoffe Wasser, Gülle und/oder Gärreste sind.

6. Verfahren nach einem der Ansprüche 1 bis 5, **gekennzeichnet dadurch, dass** zum Andicken beim Einweichen der Rohstoffe Cellulosederivate, wie Carboxymethylcellulosen zugegeben werden.

7. Verfahren nach einem der Ansprüche 1 bis 6, **gekennzeichnet dadurch, dass** als Produktionsorganismen für die Ethanolbildung Reinkulturen für die Ethanolproduktion oder auch heterofermentative Milchsäurebakterien oder Sauerteighefen verwendet werden.

## Claims

1. A method for initialising the fermentation process in biogas plants having pre-fermenters and methane fermenters using poorly biodegradable fibrous organic waste materials from agriculture which have stable fibre structures, comprising the following steps in the pre-fermenter:
a) soaking the raw materials in a liquid and heating to a temperature of 25 to 42 °C, wherein the dry substance content is a maximum of 20 % so that a stirrable pulp is produced,
b) after reaching the reaction temperature, adding enzyme-forming cultures of bacteria or fungi and cultivating for 1 to 2 days with continuous ventilation,
c) carrying out the initial transfer of hydrolysed material from the pre-fermenter to the methane fermenter and adding raw material and liquid again,
wherein the dry substance content is kept at a maximum of 20 %,
d) repeating step a) to c) a plurality of times,
e) subsequently restricting the addition of the enzyme-forming cultures to 25 to 50 % and adding production organisms for ethanol formation and cultivating for 3 to 5 days with periodic ventilation,
f) then ceasing the addition of enzyme-forming cultures and restricting the addition of ethanol-forming organisms to 25 to 50 % and cultivating for 3 to 10 days, optionally with the addition of production organisms for acetic-acid formation, wherein, as enzyme-forming cultures, microorganisms are used which form or have inherently known endo- and exocellulases and glucosidases.

2. The method according to Claim 1, **characterized by** restricting the addition of the acetic-acid forming organisms to 25 to 50 % and further cultivating for a maximum of 7 days.

3. The method according to Claim 1 or 2, **characterized in that**, when using long-fibre raw materials, the dry substance content is set to 9 -11 %,

4. The method according to any one of Claims 1 to 3, **characterized in that** the poorly biodegradable fibrous organic waste materials from agriculture are grass, grass silage, hay, straw, reed, manure.

5. The method according to any one of Claims 1 to 4, **characterized in that** the liquids for soaking the raw materials are water, slurry and/or digestates.

6. The method according to any one of Claims 1 to 5, **characterized in that** cellulose derivatives, such as carboxymethyl celluloses, are added for thickening during the soaking of the raw materials.

7. The method according to any one of Claims 1 to 6, **characterized in that** pure cultures for ethanol production or heterofermentative lactic acid bacteria or sourdough yeasts are used as production organisms for ethanol formation.

## Revendications

1. Procédé d'initialisation du processus de fermentation dans des installations de production de biogaz pourvu d'un préfermenteur et d'un fermenteur de méthanisation, au moyen de déchets organiques fibreux agricoles difficilement biodégradables, présentant des structures fibreuses stables, comprenant les étapes suivantes dans le préfermenteur :
a) trempage des matières brutes dans un liquide et chauffage à une température de 25 à 42° C, la teneur en substance sèche étant de 20 % au maximum, de manière à obtenir une bouillie susceptible d'être agitée,
b) une fois la température de réaction atteinte, ajout de cultures de bactérie ou de champignons génératrices d'enzymes, et culture pendant 1 à 2 jours avec une aération continue,
c) premier transfert de matière hydrolysée du préfermenteur vers le fermenteur de méthanisation et nouvel ajout de matières brutes et de liquide, la teneur en substance sèche étant maintenue à 20 % au maximum,
d) répétitions multiples des étapes a) à c),
e) suivie d'une limitation entre 25 et 50 % de l'ajout des cultures génératrices d'enzymes, d'un ajout d'organismes de production pour la formation d'éthanol et d'une culture pendant 3 à 5 jours avec aération discontinue,
f) ensuite, arrêt de l'ajout de cultures génératrices d'enzymes, limitation entre 25 et 50 % de l'ajout des organismes formateurs d'éthanol et culture pendant 3 à 10 jours, le cas échéant avec ajout d'organismes de production pour la formation d'acide acétique, des microorganismes étant utilisés comme cultures génératrices d'enzymes, lesquels forment ou présentent des endo- et exocellulases connues en soi ainsi que des glucosidases.

2. Procédé selon la revendication 1, **caractérisé par** limitation entre 25 et 50 % de l'ajout des organismes formateurs d'acide acétique et la poursuite de la culture pendant 7 jours au maximum.

3. Procédé selon la revendication 1 ou la revendication 2, **caractérisé en ce qu'**en cas d'utilisation de matières brutes à fibres longues, la teneur en substance sèche est ajustée entre 9 et 11 %.

4. Procédé selon l'une quelconque des revendications 1 à 3, **caractérisé en ce que** les déchets organiques fibreux agricoles difficilement biodégradables sont de l'herbe, des balles d'ensilage, du foin, de la paille, des roseaux, du fumier.

5. Procédé selon l'une quelconque des revendications 1 à 4, **caractérisé en ce que** les liquides de trempage des matières brutes sont de l'eau, du lisier et/ou des résidus de fermentation.

6. Procédé selon l'une quelconque des revendications 1 à 5, **caractérisé en ce que** des dérivés de cellulose tels que des carboxyméthylcelluloses sont ajoutés pour l'épaississement lors du trempage des matières brutes.

7. Procédé selon l'une quelconque des revendications 1 à 6, **caractérisé en ce que** des cultures pures pour la production d'éthanol ou des bactéries lactiques hétérofermentaires ou encore des levures sont utilisées en tant qu'organismes de production pour la formation d'éthanol.
